# EUROPÄISCHE PATENTSCHRIFT

(11) **EP 1 100 878 B1**
(45) Veröffentlichungstag und Bekanntmachung des Hinweises auf die Patenterteilung: **01.06.2005**
(21) Anmeldenummer: 99948706.9
(22) Anmeldetag: 22.07.1999
(51) Int. Cl.: C12N 5/10, A61K 35/14

(54) **VERFAHREN ZUR HERSTELLUNG VON GENETISCH MODIFIZIERTEN CD34-NEGATIVEN, ADHÄRENT WACHSENDEN HÄMATOPOIETISCHEN STAMMZELLEN**
PROCESS FOR PRODUCING GENETICALLY MODIFIED CD34-NEGATIVE ADHERENTLY GROWING HAEMATOPOIETIC STEM CELLS
PROCEDE POUR LA FABRICATION DES CELLULES SOUCHES HEMATOPOIETIQUES GENETIQUEMENT MODIFIEES CD34-NEGATIVES, CROISSANTES PAR ADHESION

(30) Priorität: 24.07.1998 DE 19833476
(43) Veröffentlichungstag der Anmeldung: 23.05.2001
(73) Patentinhaber: Huss, Ralf, 83666 Waakirchen (DE)
(72) Erfinder: Huss, Ralf, 83666 Waakirchen (DE)
(74) Vertreter: Grund, Martin, Dr.
(86) Internationale Anmeldenummer: PCT/DE1999/002309
(87) Internationale Veröffentlichungsnummer: WO 2000/006705

(56) Entgegenhaltungen:
- WO-A-95/02038
- US-A- 5 691 176
- HUSS ET AL: "CD34-NEGATIVE HEMATOPOIETIC STEM CELLS ISOLATED FROM HUMAN PERIPHERAL BLOOD CELLS AS ULTIMATE PRECURSORS OF HEMATOPOIETIC PROGENITORS" INFUSIONSTHERAPIE UND TRANSFUSIONSMEDIZIN, Bd. 24, 1997, Seiten 404-409, XP002130242 in der Anmeldung erwähnt
- HUSS: "APPLICATIONS OF HEMATOPOIETIC STEM CELLS AND GENE TRANSFER" INFUSIONSTHERAPIE UN TRANSFUSIONSMEDIZIN, Bd. 23, 1996, Seiten 147-160, XP000876760 in der Anmeldung erwähnt
- ROLLING F ET AL: "AAV AS A VIRAL VECTOR FOR HUMAN GENE THERAPY" MOLECULAR BIOTECHNOLOGY,US,TOTOWA, NJ, Bd. 3, Nr. 1, 1. Februar 1995 (1995-02-01), Seiten 9-15, XP002014055 ISSN: 1073-6085
- VERHASSELT ET AL: "RETROVIRALLY TRANSDUCED CD34++ HUMAN CORD BLOOD CELLS GENERATE T CELLS EXPRESSING HIGH LEVELS OF THE RETROVIRAL ENCODED GREEN FLUORESCENT PROTEIN MARKER IN VITRO" BLOOD, Bd. 91, 15. Juli 1998 (1998-07-15), Seiten 431-440, XP002130243
- ACTA HAEMATOLOGICA, Bd. 105, 2001, Seiten 159-165,
- JOURNAL OF HEMATOTHERAPY AND STEM CELL RESEARCH, Bd. 8, 1999, Seiten 335-342,

## Beschreibung

### Beschreibung der Erfindung

Die Erfindung betrifft ein Verfahren zur Herstellung von genetisch modifizierten, frühesten hämatopoetischen Stammzellen (negativ für die Expression des Oberflächenmoleküls CD34). Die Zellen sind zur Gentherapie von mono- bzw. oligogenetischen Erkrankungen, Erkrankungen der Blutbildung und auch chronischer Leiden geeignet. Dazu werden aus dem peripheren Blut des Patienten körpereigene, CD34-negative, adhärent wachsende Stammzellkulturen angelegt und diese mit Genkonstrukten effizient transfiziert bzw. infiziert. Die Genprodukte dieser Gene sollen defekte oder fehlende Proteine bzw. Faktoren im Patientenorganismus langfristig substituieren oder eine Zelltherapie möglich machen.

Das Ziel der somatischen Gentherapie bzw. der Zelltherapie ist der wirksame Transfer von genetischem Material in den Organismus. Bei der somatischen Gentherapie werden genetische Defekte in Körperzellen korrigiert oder Gene, die therapeutisch nützliche Genprodukte kodieren, in Zellen eingeschleust. Die gentherapeutische Veränderung wird dabei nicht an Nachkommen weitervererbt. Das Einschleusen von genetischem Material in Zielzellen kann *ex vivo* als auch *in vivo* vorgenommen werden. *Ex vivo* bedeutet, daß die Zielzellen außerhalb des Körpers kultiviert und nach erfolgter Einschleusung des genetischen Materials anschließend in den Patienten rücküberführt werden. Die Wirksamkeit der somatischen Gentherapie wird jedoch durch eine begrenzte Lebensdauer der transfizierten Zellen beeinträchtigt. Als Zielzellen der somatischen Gentherapie sind daher Zellen mit besonders langer Lebensdauer, wie z.B. hämatopoetische Stammzellen besonders geeignet. Bisher wurden die hämatopoetischen Stammzellen zu Transplantationszwecken entweder aus dem Knochenmark des Spenders oder nach Anreicherungsschritten aus dem peripheren Blut gewonnen. Isoliert man die Zellen aus dem Körper des Patienten oder eines nahen Verwandten, so spricht man von allogenen Knochenmarktransplantationen. Dabei wird häufig das unselektionierte Gemisch aus Stammzellen und anderen Knochenmarkzellen in den Patienten rücküberführt.

Die in diesem Gemisch enthaltenen hämatopoetischen Stammzellen wandern aus dem Blut schließlich in das blutbildende Knochenmark ein, um alle Zellen des blutbildenden Systems in notwendiger Menge zu produzieren. Eine solche Transplantation ist häufig jedoch mit schwerwiegenden Komplikationen verbunden. Durch noch so geringe Gewebeunterschiede zwischen Empfänger und Spender kann es zu lebensbedrohlichen Komplikationen für den Patienten kommen. Dieses Risiko muß mit der eigentlichen Erkrankung, z.B. Leukämie, aufgewogen werden. Die Spender-Empfänger-Unverträglichkeiten (graft versus host disease) werden im allgemeinen durch Zellen verursacht, welche das eigentliche Stammzelltransplantat verunreinigen. Es handelt sich dabei insbesondere um Zellen des Spenderimmunsystems.

Um diese Verunreinigung des Knochenmark- bzw. Stammzelltransplantats auszuschließen, wurden Verfahren entwickelt, um die Population der hämatopoetischen Stammzelle anzureichern. Diese, auch pluripotente hämatopoetische Stammzelle genannt, wird bisher über die Expression bzw. Nicht-Expression bestimmter Oberflächenmoleküle definiert. Die pluripotente hämatopoetische Stammzelle ist in der Lage, über bestimmte weitere Vorläuferzellen, die hämatopoetischen Zellinien des Menschen zu bilden, z.B. B-Zellen, T-Zellen, Leukozyten, Blutplättchen oder Erythrozyten. Die Bestimmung einer hämatopoetischen Zelle als pluripotente hämatopoetische Stammzelle wird derzeit über die Expression des sogenannten CD34-Moleküls und gleichzeitig über die Nicht-Expression anderer Oberflächenmoleküle wie CD5 bestimmt. Beim CD34-Molekül handelt es sich um ein stark negativ geladenes Proteoglykan der Muzinfamilie mit einem Molekulargewicht von etwa 105 bis 120 kD. Die Firma Cellpro, Inc., Seattle, USA, hat ein Verfahren zur Aufreinigung CD34-positiver Zellen mittels einer Affinitätschromatographie entwickelt (US-PSen 5.215.927, 5.262334, 5.240.856, 5.225.353, EP 526577 B und EP 260280 B ). Gleichzeitig bilden CD34-negative Zellen auch den Pool für mesenchymale Stammzellen.

Für die somatische Gentherapie mittels hämatopoetischer Stammzellen werden zur Zeit CD34-positive Zellen aus dem peripheren Blut der Patienten nach einer Stimulation dieser Zellen mittels Wachstumsfaktoren z.B. G-CSF (Neupogen-R) isoliert, und nach erfolgter genetischer Manipulation dem Patienten zur Rekonstitution des lethal bestrahlten und chemotherapierten Knochenmarks rücküberführt. Bisher werden derart modifizierte hämatopoetische Stammzellen insbesondere für bestimmte Immundefektsyndrome, z.B. Adenosin-Desaminase-Defekt, SCID-Syndrom oder Infektion mit HIV, für Stoffwechselerkrankungen, z.B. Morbus Gaucher, bei Blutbildungsstörungen, z.B. bestimmte Formen der Thallasämie und bei malignen Erkrankungen, z.B. Leukämien, eingesetzt.

Dieses Verfahren bleibt jedoch aus ethisch-sozialen Gründen der autologen bzw. der Blutstammzellspende durch nahe Verwandte vorbehalten, da zur Anreicherung der Stammzellen im peripheren Blut dem Spender bzw. dem Patienten ein Wachstumsfaktor gegeben werden muß, dessen Langzeitwirkung auf eine möglich Expansion eines Leukämie-Klons oder einer möglichen Transformation einer gesunden Blutstammzelle bisher nicht abgeschätzt werden konnte.

Ferner bereitet die somatische Gentherapie mittels hämatopoetischer Stammzellen derzeit noch gewisse technische Schwierigkeiten. Nur ein geringer Teil der mit therapeutischen Genen oder Genkonstrukten transfizierten hämatopoetischen Stammzellen erhält die genetische Modifikation oder es wird kein Genprodukt gebildet. Die Wirksamkeit dieses Verfahrens ist daher momentan noch sehr gering; vgl. Huss R., *Infusionsthera Transfusionsmed 23* (1996) 147-160.

Es besteht der Bedarf verbesserte Mittel und Verfahren bereitzustellen, die bei der Gentherapie eingesetzt werden können.

Die Aufgabe wird gelöst durch die in den Patentansprüchen 1 bis 8 angegebenen Gegenstände.

Gegenstand der Erfindung sind daher Verfahren zur Hestellung von genetisch modifizierten CD34-negativen, adhärent wachsenden hämatopoetischen Stammzellen.

In einer bevorzugten Ausführungsform wird die Lebenszeit bzw. Teilungsfähigkeit durch transiente Immortalisierung verlängert.

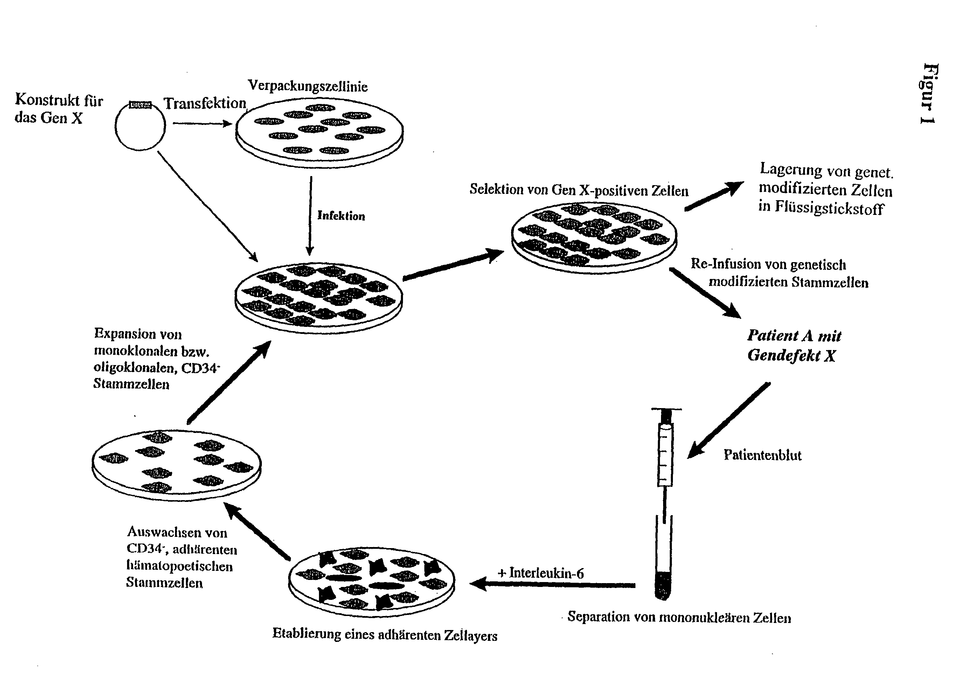

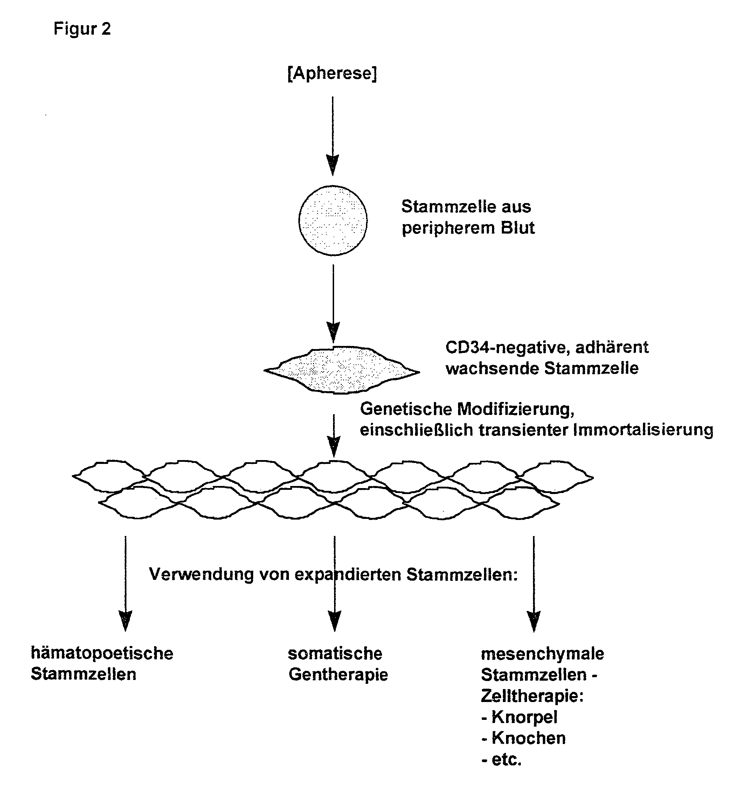

Die Erfindung wird durch die Figuren 1 und 2 und die nachfolgende Beschreibung näher erläutert.

### Detaillierte Beschreibung der Erfindung

Die initiale Beobachtung der Existenz von frühesten CD34-negativen hämatopoetischen Stammzellen beruht auf der Isolierung und Klonierung einer entsprechenden Zellinie aus primären Knochenmarkstromakulturen des Hundes. Diese bestehen aus Fibroblasten-ähnlichen, CD34-negativen Zellen, welche insbesondere in der Lage sind hämatopoetische Wachstumsfaktoren zu produzieren. Die Produktion von hämatopoetischen Wachstumsfaktoren ist bisher schon für stromale Zellinien beschrieben worden (siehe DE 4322570 C1). Aus einer solchen Primärkultur wurden in einem Standardkolonie-Assay monoklonale Populationen (Colonyforming-units = CFU) etabliert. Einige dieser Klone waren in der Lage in reifere, hämatopoetische Vorläuferzellen zu differenzieren (Huss et al., *Proc. Natl. Acad. Sci*. USA, 92:748-752; 1995). Die Differenzierung und auch die Proliferation dieser Zellen ist abhängig von verschiedenen Wachstumsfaktoren. So induziert Stammzellfaktor (c-kit ligand; SCF) die Differenzierung von CD34-negativen, adhärent wachsenden Zellen in CD34-positive, nicht mehr adhärent wachsende Zellen, während Interleukin-6 (IL-6) in erster Linie die Proliferation und das adhärente Wachstum unterstützt (Huss et al., *Blood* 85:2414-2421; 1995).

Aus CD34-positiven Zellen des peripheren Blutes konnte eine CD34-negative, adhärent wachsende, Fibroblasten-ähnliche Population etabliert werden. Es wurde gefunden, daß der Vorgang der Differenzierung vom CD34-negativen, adhärenten Wachstum zum CD34-positiven, nicht mehr adhärenten Wachstum auch reversibel war. Durch die Zugabe von IL-6 wurde aus peripheren, mononukleären Zellen von gesunden, freiwilligen Spendern eine adhärente, jedoch zunächst nicht homogene Zellpopulation etabliert. Zu diesem Zweck wurden ca. 20 ml hepariniertes Blut zweifach über einen Ficoll-Gradienten gegeben, die mononukleäre Zellfraktion nach Standardmethoden isoliert und in IL-6 haltigem Medium in Zellkulturflaschen in einen Inkubator bei 37 °C und 5% CO₂ gegeben. Nach wenigen Tage entstand ein adhärent wachsender Zellrasen. Diese Primärkultur bestand zunächst aus den Fibroblasten-ähnlichen Zellen, sowie Makrophagen, Endothelzellen und vereinzelt Fettzellen. Dies wurde mittels Immunphänotypisierung nachgewiesen. Jedoch nahm die Anzahl der "kontaminierenden" Zellen im Verlauf der ersten Tage und Wochen vollständig ab (Huss et al., *Infusionsthera Transfusionsmed* 24:404-409; 1997), bis eine nahezu homogene Zellpopulation entstanden war. Jedoch ist diese Population zu diesem Zeitpunkt nicht klonierbar, da diese nicht immortalisiert wurde. Dennoch können diese Zellen entweder in der Zellkultur gehalten oder in üblicher Weise in Flüssigstickstoff eingefroren werden.

Durch Modifikationen der Bedingungen in der Zellkultur können CD34-negative, adhärent wachsende Zellen auch in mesenchymale Stammzellen differenzieren, und somit eine Vorläuferzelle für Knochen, Knorpel und andere Gewebe bilden.

### Gentransfer

Es wurden CD34-negative, adhärent wachsende, Fibroblasten-ähnliche Zellen aus aufgereinigten, mononukleären Zellen des peripheren Blutes nach vorstehendem Verfahren isoliert. Zunächst war die Kontamination mit anderen Zellen sehr hoch, welche jedoch mit der Zeit in Kultur vollständig verschwanden, sodaß 100% der adhärenten Zellen als CD34-negative, adhärente Zellen vorlagen. Um die Transduktionseffizienz optimal zu gestalten, wurden verschiedene Methoden zum Gentransfer von "green-fluorescence protein" (GFP) untersucht. Beim "green-fluorescence-protein" handelt es sich um ein Genkonstrukt, welches in transfizierten bzw. infizierten Zellen unter ultraviolettem Licht grün aufleutet und so den Nachweis einer mit GFP transfizierten bzw. infizierten Zelle auf einfache Art und Weise ermöglicht.

Die Ergebnisse mit in-vitro Kulturen als auch in-vivo Versuche in SCID-Mäusen [-*SCID-Mäuse besitzen einen zellulären Immundefekt, wodurch sich diese Mäuse als in-vivo Modell für allogene oder xenogene Transplantationsmodelle eignen*-] haben gezeigt, daß die Expression von GFP in den Zielzellen viele Wochen andauert, wobei die Fluoreszenz von GFP-transfizierten Zellen in SCID-Mäusen zwar deutlich sichtbar ist, jedoch schwächer ist als im reinen Zellkulturverfahren.

Die Untersuchung verschiedener Transfektions- bzw. Infektionsmethoden für das GFP-Protein erbrachte unterschiedliche Ergebnisse:

Die CaCl₂-Methode zur Transfektion induzierte einen starken Zellstress, sodaß ein großer Teil der zu transfizierenden Zellen abstarben.

Dagegen war die Effizienz des Gentransfers mittels Lipofectamin bzw. Infektion mit viralem Überstand sehr hoch. Während die Transfektionseffizienz mit Lipofectamin nach einmaliger Anwendung bei ca. 40-50% lag, war die Infektionseffizienz mit viralem Überstand nach 10 bis 14 Tagen bei 3-4 Passagen bei 88-94%. Entsprechend wurde alle 3-4 Tage neuer, virushaltiger Überstand auf die Zielzellen gegeben.

**Tabelle:**

| | % GFP-positiv | Wiederholung | % tote Zellen |
|---|---|---|---|
| Transfektion mit CaCl₂ | 68 ± 17 | nein | 40 ± 21 |
| Transfektion mit Lipofectamin | 47 ± 6 | nein | < 5 |
| Infektion mit viralem Überstand | 91 ± 5 | ja | < 1% |

### Effizienz

Der unübersehrbare Vorteil des erfindungsgemäßen Systems liegt in der hohen Transfektions- bzw. Infektionseffizienz dieser sehr homogenen, wenn auch meist noch nicht monoklonalen, so doch meistens oligoklonalen Zellpopulation.

Während ähnliche Verfahren trotz intensiver Bemühungen bei einer Infektionseffizienz von ca. 5-20% von hämatopoetischen Vorläuferzellen liegen, sind nahezu alle Zellen der erfindungsgemäßen frühen Stammzellpopulation mit dem gewünschten Gen infiziert.

Versuche in Zusammenarbeit mit SCID-Mäusen haben gezeigt, daß die Expression des Gens in allen Reihen der Hämatopoese auch in-vivo erfolgt. Dies gilt nicht nur für retrovirale Konstrukte, sondern auch für die Anwendung Adeno-virus assoziierter Viren (AAV) oder Konstrukte, welche auf dem Epstein-Barr-Virus (EBV) beruhen.

### Homogenität

Die adhärent wachsenden, Fibroblasten-ähnlichen, nahezu homogenen Zellpopulation lassen sich aufgrund der leichten Isolierbarkeit aus peripherem Blut und der hohen Effizienz hervorragend im Bereich der Gentherapie und Zelltherapie einsetzen.

Experimente haben gezeigt, daß diese frühesten hämatopoetischen Stammzellen nicht nur langfristig rekonstituieren können, sondern über die Besiedlung des Thymus auch eine Toleranz induzieren können.

Dies erlaubt eine Anwendung nicht nur im autologen oder syngenen System, sondern auch im Bereich der allogenen Transplantation.

### Therapieoptionen

Alle Gene, die im Patienten ein defektes oder nicht vorhandenes Genprodukt repräsentieren, können so durch die Infektion von autologen, hämatopoetischen Zellen mit einem Konstrukt für das fehlende oder defekte Gen dem Patienten mit seinen körpereigenen, genetisch modifizierten Zellen wieder zugeführt werden (siehe Figur 1).

Kandidaten sind insbesomdere Gene bei bestimmten metabolischen Stoffwechsel-erkrankungen (z.B. M. Gaucher, PNH, Diabetes mellitus), bei Immundefektsyndromen (ADA-Mangel, SCID, CGD, LAD, AIDS), Hämoglobinopathien (z.B. Sichelzellanämie, Thalassämie) und malignen Erkrankungen (z.B. MDR, Antisense-Konstrukte, Hammerhead-Ribozyme bei identifizierten Mutationen)
[*siehe auch Tabelle 1, Seite 8: Somatic Gene Therapy; P.L. Chang* (ed.) CRC Press, Inc., Boca Raton, USA].

Die vom Patienten isolierten Zellen können nach dem Transfer des gewünschten Gens in die autologen, frühen hämatopoetischen Stammzellen des Patienten auch in Flüssigstickstoff tiefgefroren werden, um sie später bei Bedarf erstmals oder nochmals zu verwenden. Dies ist möglich, da diese Zellen sehr ausgiebig proliferieren.

Ein weitere Ausführungsform betrifft den Einbau eines "Suizid-Gens", z.B. der Thymidin-Kinase, um ggf. mittels Ganciclovir diese infizierten Zellen komplett oder teilweise eliminieren zu können. Dies ermöglicht die Zellen auch in-vivo weiterhin unter einer Wachstumskontrolle zu behalten, z.B. wenn die Aktivität eines Enzyms den gewünschten Serumspiegel übersteigen würde oder die Tumorerkrankung ausgeheilt ist.

Die teilweise transient (oder passager) immortalisierten Stammzellen können auch für eine Zelltherapie oder zur Herstellung von körpereigener Knorpel- bzw. Knochensubstanz verwendet werden (siehe Figur 2).

Bei dieser Ausführungsform ist es ebenfalls nicht nötig, den Patienten einer myeloablativen Therapie zu unterziehen, da der Patient nicht vollständig hämatopoetisch rekonstituiert werden muß, sondern nur ein Teil der hämatopoetischen Stammzellen durch die genetisch modifizierten Stammzellen ersetzt wird.

Die nachfolgenden Beispiele erläutern die Erfindung

### Beispiel 1: Isolierung von Zellen

Dem Patienten bzw. freiwilligen Spender werden ca. 20 ml hepariniertes Blut oder Citratblut abgenommen und über einen Ficoll-Hypaque Gradienten (Pharmacia Biotech, Uppsala, Schweden) geschichtet (siehe auch Huss et al., *Infusionsthera Transfusionsmed* 24:404-409, 1997).

Nach 15 - 20 min Zentrifugation bei 400 x g ohne Bremse wird der Ring aus mononukleären Zellen abgenommen und mehrfach in PBS oder isotonischer Kochsalzlösung gewaschen.

Anschließend werden 5x10⁶ bis 1x10⁷ Zellen / ml in einer Zellkulturflasche (z.B. NUNC) in Zellkulturmedium (z.B. McCoy's Standard Medium mit 12,5% fötalem Kälberserum und 12,5% Pferdeserum) in einem Inkubator bei 37°C und 5% CO₂ in Gegenwart von 10 ng/ml rekombinantem Interleukin-6 (rhu IL-6; RD Systems GmbH, Wiesbaden) inkubiert. Die Kulturen werden täglich beobachtet und wöchentlich gezählt.

Nach 10-14 Tagen entsteht eine homogene Zellpopulation aus adhärent wachsenden, Fibroblasten-ähnlichen Zellen, welche bei der Analyse im FACScan bzw. in der Immunhistochemie nahezu volständig CD34-negativ sind. Einige, differenzierende Zellen exprimieren zeitweise dennoch das CD34-Antigen, da diese Zellen nicht synchronisiert sind.

### Beispiel 2: Retrovirale Infektion / am Beispiel der Infektion mit EBV-Konstrukten

Diese oben beschriebene homogene Zellpopulation wird nun mit retroviralem Überstand der PG-13 Zellinie (*Eine Verpackungszelline, welche den gewünschten retroviralen Vektor nach Transfektion der PG-13 Zellen in eine Gibbonaffen-Leukämievirushülle verpackt. Die Transfektion der Verpackungszellinie geschieht indem 2,5 µg Plamid-DNA mit 15 µl Superfect (Qiagen) gemischt werden und in 100 µl Serum-freiem Medium bei Raum-Temperatur für 20 Minuten inkubiert werden. Anschließend wird diese Mischung für 5 Stunden bei 37°C mit den PG-13 Zellen inkubiert, bevor erneut Serum-haltiges Medium zugegeben wird*.) über einen Zeitraum von ebenfalls 10-14 Tagen inkubiert, wobei das Virus-haltige Medium mindestens 3-4 mal gewechselt werden sollte. Nach dieser Zeit wird die Genexpression (bei GFP mittels Fluoreszenzmikroskopie) in den Zielzellen untersucht und die Effizienz festgestellt.

Die positiven Zellen können nun dem Patienten zurückgegeben oder für einen späteren Gebrauch eingefroren werden. Bei diesen retroviralen Infektionen liegt die MOI (*Multiplicity of Infection = wieviel Viruspartikel sind für die Infektion einer Zelle erforderlich*) bei 10.

Die Zellen können auch transient immortalisiert werden, um eine Expansion der Stammzellen zu erreichen.

### Beispiel 3: Infektion der aus dem Patienten isolierten Zielzellen mit rekombinantem AAV-Virus, welcher Luziferase exprimiert

Die aus dem Patienten isolierten Zielzellen konnten sehr effizient ebenfalls mit rekombinantem AAV-Virus infiziert werden, welcher Luziferase exprimiert. Die Zielzellen des Patienten wurden für 3 Stunden bei 37°C mit LUC-rAAV inkubiert und anschließend für weitere 72 Stunden in Serum-haltigem Medium gehalten. Anschließend wurde die Luziferase-Aktivität als auch die Lumineszenz gemessen. In diesem System liegt die MOI bei 1000.

### Beispiel 4: Patientenbeispiel 1

Die Vorgehensweise ist schematisch in Figur 1 dargestellt.

Dem Patienten A mit dem Gendefekt X wird aus der Armvene Blut entnommen und aus diesem periphere mononukleäre Zellen mittels Gradientenzentrifugation isoliert. Diese mononukleären Zellen werden in der Zellkultur mit Interleukin-6 inkubiert bis sich ein adhärenter Zellrasen etabliert hat.

In der primären Kultur ist die Kontamination mit nicht hämatopoetsichen Stammzellen noch sehr hoch. Nach 2-4 Wochen sind nahezu ausschließlich CD34-negative, hämatopoetische Stammzellen ausgewachsen, welche nun expandiert werden können.

Diese werden nun mit retroviralen Viruskonstrukten oder AAV-Viren, welche zuvor mit dem gewünschten Genkonstrukt transfiziert worden waren, infiziert. Nach wenigen Tagen kann in den hämatopoetischen Stammzellen des Patienten A nun die Gegenwart des Gens X und die Expression des Genproduktes X (z.B. Glucocerebrosidase oder Insulin) nachgewiesen werden.

Nun erhält der Patient A seine genetisch modifizierten, frühen hämatopoetischen Stammzellen über eine Infusion zurück, wobei ein Teil dieser Zellen zur späteren Verwendung in Flüssigstickstoff konserviert wird. Die nun mit einer neuen genetischen Information versehen Blutstammzellen des Patienten versorgen den Patienten A nun mit dem für ihn wichtigen Genprodukt X.

Im Falle des Diabetes mellitus wird es schon reichen einen ganz geringen Prozentsatz der körpereigenen Stammzellen mit dem neuen genetischem Material zu versehen, um den Blutzuckerspiegel langfristig zu senken und damit ebenfalls den notwendigen Insulinbedarf. Dies würde bei Diabetespatienten sicherlich auch die schweren Beleiterkrankungen langfristig verzögern oder gar verhindern.

### Beispiel 5: Patienten beispiel 2

Patent B leidet an einem schweren Knorpeldefekt im Kniegelenk. Dem Patienten werden periphere Blutstammzellen entnommen (z.B. durch Apherese) und transiert (passager) immortalisiert (z.B. durch SV40- Large-T Antigen in einem cre/lox-System mit EBNA1). Diese Zellen werden dann in der Zellkultur in Knorpel-/ Knochenvorläuferzellen differenziert und an die defekte Stelle transplantiert.

## Patentansprüche

1. Verfahren zur Herstellung von genetisch modifizierten CD34-negativen, adhärent wachsenden hämatopoetischen Stammzellen umfassend die Schritte:
(i) Inkubation von isolierten peripheren mononukleären Zellen mit Interleukin-6, wobei eine homogene Zellpopulation aus adhärent wachsenden, Fibroblasten-ähnlichen Zellen entsteht, die nahezu vollständig CD34-negativ sind;
(ii) Transfektion der homogenen Zellpopulation mit einem therapeutischen Gen; und
(iii) Isolierung von positiv transfizierten CD34-negativen, adhärent wachsenden hämatopoetischen Stammzellen.

2. Verfahren gemäß Anspruch 1, wobei die Zellen immortalisiert werden, um eine Expansion der Stammzellen zu erreichen.

3. Verfahren gemäß Anspruch 1, wobei das therapeutische Gen für Insulin, Faktor VIII, Adenindesaminase, Multidrug-Resistance (MDR) oder einen Immortalisierungsfaktor codiert.

4. Verfahren gemäß Anspruch 1, wobei die mononukleären Zellen aus heparinisiertem Blut oder Citrat-Blut von Patienten stammen.

5. Verfahren gemäß einem der Ansprüch 1-4, wobei die Transfektion in Schritt (ii) mittels der CaCl₂-Methode oder Lipofectamin erfolgt.

6. Verfahren gemäß einem der Ansprüche 1-4, wobei die Transfektion in Schritt (ii) mittels eines retroviralen Konstrukts oder eines EBV-Konstrukts oder eines Adenovirus-assoziierten Konstrukts erfolgt.

7. Verfahren gemäß einem der Ansprüche 1-4, wobei die Transfektion mit einem retroviralen Überstand der PG-13 Zelllinie erfolgt.

8. Verfahren gemäß einem der Ansprüche 1-7, wobei es sich bei dem eingesetzten Interleukin-6 um rekombinantes Interleukin-6 handelt.

## Claims

1. A method for producing genetically modified CD34-negative adherently growing haematopoietic stem cells comprising the steps of:
(i) incubation of isolated peripheral mononuclear cells with interleukin-6, wherein a homogenous cell population is generated from growing fibroblast-like cells that are almost completely CD34-negativ;
(ii) transfection of the homogenous cell population with a therapeutic gene; and
(iii) isolation of positively transfected CD34-negative adherently growing haematopoietic stem cells.

2. The method according to claim 1, wherein the cells are immortalized to obtain an expansion of the stem cells.

3. The method according to claim 1, wherein the therapeutic gene encodes for insulin, factor VIII, adenine desaminase, multidrug resistance (MDR) or an immortalizing factor.

4. The method according to claim 1, wherein the mononuclear cells originate from heparinized blood or citrate blood of patients.

5. The method according to any one of claims 1-4, wherein the transfection in step (ii) is carried out by the CaCl₂ procedure or lipofectamine.

6. The method according to any one of claims 1-4, wherein the transfection in step (ii) is carried out by means of a retroviral construct or an EBV construct or an adenovirus-associated construct.

7. The method according to any one of claims 1-4, wherein the transfection is carried out with a retroviral supernatant of the cell line PG-13.

8. The method according to any one of claims 1-7, wherein the utilized interleukin-6 is recombinant interleukin-6.

## Revendications

1. Procédé pour la préparation de cellules souches hématopoïétiques génétiquement modifiées CD34-négatives, croissantes par adhésion, comprenant les étapes :
(i) incubation de cellules mononucléaires périphériques isolées avec l'interleukine 6, par laquelle se forme une population cellulaire homogène de cellules analogues à des fibroblastes, croissantes par adhésion, qui sont presque complètement CD34-négatives ;
(ii) transfection de la population cellulaire homogène avec un gène thérapeutique ; et
(iii) isolement de cellules souches hématopoïétiques transfectées positives, CD34-négatives, croissantes par adhésion.

2. Procédé selon la revendication 1, dans lequel les cellules sont immortalisées, pour obtenir une expansion des souches cellulaires.

3. Procédé selon la revendication 1, dans lequel le gène thérapeutique code pour l'insuline, le Facteur VIII, l'adénine désaminase, la résistance multidrogues (MDR, Multidrug-Resistance) ou un facteur d'immortalisation.

4. Procédé selon la revendication 1, dans lequel les cellules mononucléaires sont issues de sang hépariné ou de sang citraté de patients.

5. Procédé selon l'une des revendications 1 à 4, dans lequel on effectue la transfection à l'étape (ii) au moyen de la méthode CaCl₂ ou de lipofectamine.

6. Procédé selon l'une des revendications 1 à 4, dans lequel on effectue la transfection à l'étape (ii) au moyen d'un gène chimérique rétroviral ou d'un gène chimérique de V.E.B. ou d'un gène chimérique associé à un adénovirus.

7. Procédé selon l'une des revendications 1 à 4, dans lequel on effectue la transfection avec un surnageant rétroviral de la lignée cellulaire PG-13.

8. Procédé selon l'une des revendications 1 à 7, dans lequel il s'agit pour l'interleukine 6 d'interleukine 6 recombinante.
